(19) Europäisches Patentamt
European Patent Office
Office européen des brevets

(11) Publication number : **0 445 920 A2**

(12) **EUROPEAN PATENT APPLICATION**

(21) Application number : **91300951.0**

(22) Date of filing : **06.02.91**

(51) Int. Cl.$^5$ : **A61K 31/165**, A61K 31/19, A61K 31/64

(30) Priority : **13.02.90 GB 9003185**

(43) Date of publication of application : **11.09.91 Bulletin 91/37**

(84) Designated Contracting States : **AT BE CH DE DK ES FR GB GR IT LI LU NL SE**

(71) Applicant : **IMPERIAL CHEMICAL INDUSTRIES PLC**
**Imperial Chemical House, Millbank**
**London SW1P 3JF (GB)**

(72) Inventor : **Holloway, Brian Roy**
**64 Bailey Crescent, Buglawton**
**Congleton, Cheshire, CW12 2EW (GB)**
Inventor : **Stribling, Donald**
**Aldersey Fold, London Road, Prestbury**
**Nr Macclesfield, Cheshire (GB)**

(74) Representative : **Denerley, Paul Millington, Dr. et al**
**Imperial Chemical Industries PLC Legal Department: Patents P.O. Box 6, Bessemer Road**
**Welwyn Garden City Hertfordshire AL7 1HD (GB)**

(54) **Pharmaceutical compositiobns.**

(57) Pharmaceutical compositions containing 4-[2-(2-hydroxy-3phenoxypropylamino)ethoxy]-N-(2-methoxyethyl)phenoxyacetamide or it's acid metabolite or pharmaceutically acceptable salts thereof and a sulphonylurea are described. Such compositions are useful in the treatment of diabetes mellitus and related conditions.



Jouve, 18, rue Saint-Denis, 75001 PARIS

# PHARMACEUTICAL COMPOSITIONS

The present invention relates to pharmaceutical compositions and in particular to pharmaceutical compositions containing 2-(phenoxypropanolamino)ethoxyphenoxyacetic acid derivatives. These compositions are useful in the therapeutic treatment of animals including humans in particular in the treatment of diabetes mellitus and related disease conditions.

Diabetes mellitus is a metabolic dysfunction accompanied by elevated glucose levels in the blood. Therapeutic treatment may be effected in a variety of ways, for example using insulin, dietary control (for obese adults) or using oral hypoglycaemic agents such as biguanides or sulphonylureas.

A side-effect of the use of sulphonylureas is that they can cause weight gain in patients under treatment (Clarke and Campbell, British Medical Journal, 275, 1576-8 (1977); Clarke and Duncan, Lancet; i, 123-6 (1968)). This is undesirable as control of body weight and prevention of obesity is an important factor in the treatment of diabetes mellitus. It has been stated that because of this propensity for sulphonylureas to promote weight gain, metformin has a major advantage in terms of preventing weight gain or possibly promoting weight loss.

The combination of a specific small class of 2-(phenoxypropanolamino)ethoxyphenoxyacetic acid compounds with a sulphonylurea lessens the problems associated with the use of sulphonylureas. In particular the obesifying effects of sulphonylureas are diminished; control of triglyceride levels is improved with propensity to raise HDL cholesterol levels; and furthermore the compositions of the present invention are useful for the control of glycaemia when a sulphonylurea alone is not adequate.

Accordingly the present invention provides a pharmaceutical composition which comprises a compound of the formula (I):

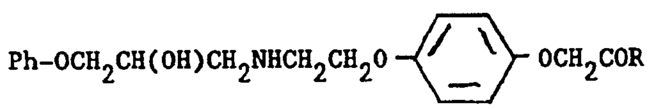 (I)

wherein R is hydroxy or 2-methoxyethylamino or a pharmaceutically acceptable salt thereof and a sulphonylurea.

Preferably in the compounds of the formula (I) R is 2-methoxyethylamino.

The compounds of the formula (I) contain an asymmetric carbon atom and can exist as an optically active enantiomer (R or S according to the Cahn-Ingold-Prelog convention) or as an optically inactive racemate. The compounds for use in the present invention contain at least some of the laevorotatory optically active form (-) which corresponds to (S) absolute configuration. Conveniently the compounds for use in the present invention are provided as the racemate but preferably are provided as the laevorotatory optically active form (-).

The compounds of formula (I) are basic and may be isolated and used either in the form of a free base or of a pharmaceutically acceptable acid-addition salt thereof. In addition, the compound of the formula (I) wherein R is hydroxy is amphoteric and may be isolated and used in the zwitterionic form, or as a pharmaceutically acceptable acid-addition salt, or as a salt with a base affording a pharmaceutically acceptable cation.

Particular examples of pharmaceutically acceptable acid-addition salts include, for example, salts with inorganic acids such as hydrohalides (especially hydrochlorides or hydrobromides), sulphates and phosphates, and salts with organic acids such as the free acid form of sulphonated polystyrene.

Particular examples of salts with bases affording a pharmaceutically acceptable cation include, for example, alkali metal and alkaline earth metal salts, such as sodium, potassium, calcium and magnesium salts, and ammonium salts and salts with suitable organic bases, such as triethanolamine.

A preferred compound for use in the methods of the present invention is:(S)-4-(2-(2-hydroxy-3-phenoxypropylamino)ethoxy]-N-2-methoxyethyl)phenoxyacetamide hydrochloride.

The compound of the formula (I) and pharmaceutically acceptable salts thereof wherein R is hydroxy are known from, and/or can be prepared by, the methods of European Patent Application Publication No 210849. The compounds of the formula (I) and pharmaceutically acceptable salts thereof wherein R is 2-methoxyethylamino are known from, and/or can be prepared by, the methods of European Patent Application Publication No 254532.

The compounds of the formula (I) and their pharmaceutically acceptable salts are known as thermogenic agents, that is they stimulate thermogenesis in warm-blooded animals and are of use, for example, in the treatment of obesity and related conditions, such as obesity related to mature onset diabetes. These compounds may also be of value in the modification of carcass composition, for example, by increased catabolism of fat

in meat producing animals, such as cattle, pigs, sheep, goats and/or rabbits.

The sulphonylureas useful in the compositions of the present invention are those useful as hypoglycaemic agents. Suitable sulphonylureas include acetohexamide, chlorpropamide, glibenclamide, glibornuride, gliclazide, glipizide, gliquidone, glisoxepide, tolazamide and tolbutamide. Preferred sulphonylureas are acetohexamide, chlorpropamide, glibenclamide and tolbutamide, in particular glibenclamide.

The pharmaceutical compositions of this invention may be administered in standard manner for example by oral or parenteral administration. For these purposes they may be formulated by means known to the art into the form of, for example, tablets, capsules, pills, powders, aqueous or oily solutions or suspensions, emulsions, and sterile injectable aqueous or oily solutions or suspensions.

In particular compositions for oral administration are preferred, as the sulphonylureas and compounds of the formula (I) are both active by the oral route.

The compositions may be obtained using standard excipients and procedures well known in the art. A unit dose formulation such as a tablet or capsule will usually contain, for example 0.1-250mg of a compound of the formula (I) or a pharmaceutically acceptable salt thereof and 1-500mg of a sulphonylurea. Preferably a unit dose formulation will contain 1-100mg of a compound of the formula (I) or a pharmaceutically acceptable salt thereof and 5-500mg of a sulphonylurea.

The pharmaceutical compositions of this invention may be administered up to six times daily, conveniently 1 to 4 times daily, so that a dose of the compound of the formula (I) or pharmaceutically acceptable salt thereof in the general range 0.002-20mg/kg, preferably 0.02-10mg/kg, is administered daily and a dose of sulphonylurea in the general range 0.02-60mg/kg, preferably 0.1-20mg/kg, is administered daily. It will be appreciated by those skilled in the art that the dosage will necessarily be varied as appropriate, according to the severity of the condition under treatment, according to the age and sex of the patient and according to known medical principles. In addition, account should be taken of the recommended maximum daily dosages for the sulphonylureas; for example for glibenclamide, chlorpropamide, acetohexamide and tolbutamide these are in the regions 15mg, 500mg, 1.5g and 1.5g respectively.

The present invention covers the combination of a compound of the formula (I) or pharmaceutically acceptable salt thereof and a sulphonylurea for simultaneous, separate or sequential use in therapy for example diabetic therapy. In one aspect the compound of the formula (I) or pharmaceutically acceptable salt thereof and sulphonylurea are presented in admixture in one pharmaceutical dosage unit. In another aspect, the present invention covers the administration of separate unit dosages of the compound of the formula (I) and sulphonylurea in order to achieve the desired therapeuticeffect. Such separate unit dosages may be administered concurrently or sequentially as determined by the clinician. Suitable separate unit dosages may be those used conventionally for the administered compound.

The following biological data and examples serve to illustrate the present invention.

Effects of (S)-4-[2-(2-hydroxy-3-phenoxypropylamino)ethoxy]-N-(2-methoxyethyl)phenoxyacetamide on glibenclamide induced improvements in oral glucose tolerance in rats

Glibenclamide is a sulphonylurea commonly used for the treatment of non-insulin dependent diabetes. The effects of (S)-4-[2-(2-hydroxy-3-phenoxypropylamino)ethoxy]-N-(2-methoxyethyl) phenoxyacetamide (compound A) were assessed on glibenclamide induced improvements in oral glucose tolerance.

Method

Male, Alderley Park, Wistar rats (weight range 125-150 g) were caged in groups of four in a room with normal lighting, at a temperature of 22°C. The animals were allowed free access to water and chow during an acclimatisation period of five days.

At the end of this period the rats were fasted for 24 hours. After fasting a group of 8 rats was anaesthetised and a cardiac blood sample taken. The remaining rats were dosed orally according to the schedule below:

| Treatment | Time relative to oral glucose load (hours) | |
|---|---|---|
| | -2.0 | -0.5 |
| Control | Excipient | Excipient |
| Glibenclamide treated | Glibenclamide (5 mg/kg) | Excipient |
| Compound A treated | Excipient | Compound A (0.1 mg/kg) |
| Glibenclamide + Compound A treated | Glibenclamide (5 mg/kg) | Compound A (0.1 mg/kg) |

Glibenclamide and Compound A were dissolved in an aqueous 0.025% solution of polysorbate. The dosage volume was 0.5 ml/100 g body weight.

Thirty minutes after dosing according to the schedule above a further eight rats from each group were bled. The remaining rats were then given an oral glucose load (1g/kg) dosed as a 20% solution of D-glucose (0.5 ml/100 g). Groups of eight rats for each treatment were then anaesthetised and bled 20, 60 and 120 minutes after the glucose load. Glucose levels were assayed using a glucose oxidase method on a KDK glucose analyser (Clandon).

Results

The comparative effects of treatment with Compound A, glibenclamide and Compound A plus glibenclamide are summarised in the following Table.

Table Effects of Compound A on glibenclamide induced improvements in oral glucose tolerance in Alderley Park rats.

| Time relative to oral glucose (minutes) | -30 | 0 | 20 | 60 | 120 |
|---|---|---|---|---|---|
| Controls | 4.25 ± 0.72 | 3.86 ± 0.17 | 8.85 ± 0.29 | 7.64 ± 0.15 | 5.65 ± 4.36 |
| Glibenclamide (5 mg/kg p.o.) | - | 3.16 ± 0.13 ** | 7.77 ± 0.45 | 4.4 ± 0.38 *** | 3.67 ± 0.27 *** |
| Compound A (0.1 mg/kg p.o.) | - | 2.77 ± 0.10 *** | 6.16 ± 0.23 | 5.37 ± 0.28 *** | 4.99 ± 0.20 |
| Glibenclamide (5 mg/kg p.o.) plus Compound A (0.1 mg/kg p.o.) | - | 2.10 ± 0.15 | 4.90 ± 0.33 | 3.26 ± 0.32 *** | 3.17 ± 0.35 *** |

Results are M ± SEM of observation in eight rats/group and are expressed in mmoles
Significant differences from control: *P<0.05, **P<0.01, ***P<0.001

Examples 1-3

Suitable pharmaceutical compositions of the compounds of the formula (I) and sulphonylureas may be obtained by standard formulation techniques, for example by direct compression or, if necessary, by aqueous granulation followed by compression.

Particular pharmaceutical compositions include the following tablets:

| Example 1 | mg |
|---|---|
| Glibenclamide | 5 |
| Compound A | 25 |
| Lactose | 150 |
| Maize starch | 20 |
| Magnesium stearate | 2 |

| Example 2 | |
|---|---|
| Tolbutamide | 500 |
| Compound A | 25 |

| | |
|---|---|
| Maize starch | 50 |
| Magnesium stearate | 5 |
| Example 3 | |
| Chlorpropamide | 250 |
| Compound A | 25 |
| Lactose | 100 |
| Maize starch | 50 |
| Magnesium stearate | 5 |

Example 4

A patient requiring treatment for diabetes mellitus is treated with (S)-4-[2-(2-hydroxy-3-phenoxypropylamino)ethoxy]-N-(2-methoxyethyl)-phenoxyacetamide hydrochloride (150mg) and with glibenclamide (5mg). Each compound is administered twice daily.

## Claims

1. A pharmaceutical composition which comprises a compound of the formula (I):

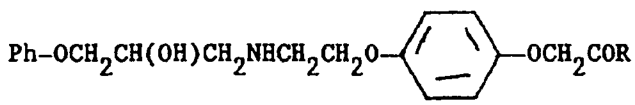

(I)

wherein R is hydroxy or 2-methoxyethylamino or a pharmaceutically acceptable salt thereof and a sulphonylurea.

2. A composition according to claim 1 wherein R is 2-methoxyethylamino.

3. A composition according to claim 1 wherein the compound of the formula (I) is (S)-4-[2-(2-hydroxy-3-phenoxypropylamino)ethoxy]-N-(2-methoxyethyl)phenoxyacetamide hydrochloride.

4. A composition according to any one of claims 1 to 3 wherein the sulphonylurea is acetohexamide, chlorpropamide, glibenclamide or tolbutamide.

5. A composition according to claim 4 wherein the sulphonylurea is glibenclamide.

6. A composition according to any one of claims 1 to 5 in a form suitable for oral administration.

7. A composition according to any one of claims 1 to 6 for use in a method of therapy.

8. The use of a compound of the formula (I):

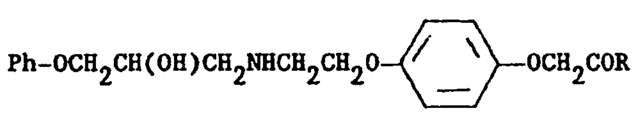

(I)

wherein R is hydroxy or 2-methoxyethylamino or a pharmaceutically acceptable salt thereof and a sulphonylurea in the manufacture of a medicament for treating diabetes mellitus and related conditions.

9. A process preparing a pharmaceutical composition which comprises bringing into association a compound of the formula (I) as defined in claim 1 or a pharmaceutically acceptable salt thereof and a sulphonylurea.